(19) Europäisches Patentamt
European Patent Office
Office européen des brevets

(11) Veröffentlichungsnummer: **0 394 982 A2**

(12) **EUROPÄISCHE PATENTANMELDUNG**

(21) Anmeldenummer: **90107812.1**

(51) Int. Cl.⁵: **C07H 15/252, A61K 31/70**

(22) Anmeldetag: **25.04.90**

(30) Priorität: **26.04.89 DE 3913742**

(43) Veröffentlichungstag der Anmeldung: **31.10.90 Patentblatt 90/44**

(84) Benannte Vertragsstaaten: **AT BE CH DE DK ES FR GB GR IT LI LU NL SE**

(71) Anmelder: **BEHRINGWERKE Aktiengesellschaft**
**Postfach 1140**
**D-3550 Marburg 1(DE)**

(72) Erfinder: **Hermentin, Peter, Dr.**
**Salzköppel 9**
**D-3550 Marburg(DE)**
Erfinder: **Raab, Ernst**
**Weidenbrunkel 13**
**D-3550 Marburg(DE)**
Erfinder: **Kraemer, Hans Peter, Dr. Dr.**
**Birkenweg 16**
**D-3550 Marburg(DE)**
Erfinder: **Czech, Jörg, Dr.**
**Höhenweg 3**
**D-3550 Marburg(DE)**

(74) Vertreter: **Klein, Otto, Dr. et al**
**Hoechst AG Zentrale Patentabteilung**
**Postfach 80 03 20**
**D-6230 Frankfurt am Main 80(DE)**

(54) **Zytostatisch wirksame Rhodomycinderivate.**

(57) Die Erfindung betrifft neue, zytostatisch wirksame Anthracyclinderivate der Formel I

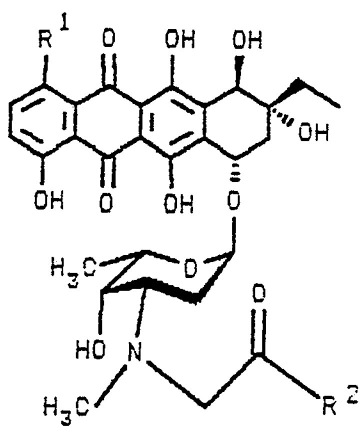

I

und deren physiologisch unbedenklichen Salze, wobei $R^1$ Wasserstoff oder eine Hydroxygruppe und $R^2$ $NR^a-(CH_2)_n-NR^aR^b$ mit n = 0-12 ist, wobei $R^a$ und $R^b$ Wasserstoff, $C_1-C_4$-Alkyl oder Benzyl sind, oder $R^2$ $NR^a-CHR^3-CH_2-R^4$ ist, wobei $R^a$ die genannte Bedeutung hat und $R^3$ Wasserstoff oder eine Carboxylgruppe und $R^4$ der Rest einer L-Aminosäure oder eines biogenen Amins ist, ein Verfahren zu ihrer Herstellung und ihre Verwendung in einem Arzneimittel.

EP 0 394 982 A2

## Zytostatisch wirksame Rhodomycinderivate

Die Erfindung betrifft neue, zytostatisch wirksame Anthracyclinderivate der Formel I und deren physiologisch unbedenklichen Salze, Verfahren zu ihrer Herstellung sowie ihre Verwendung als Arzneimittel.

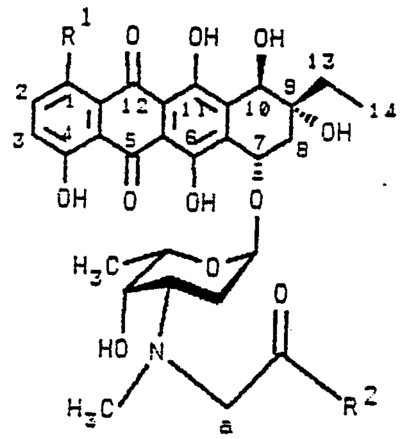

I

Für Formel I gilt:
$R^1$ ist Wasserstoff oder eine Hydroxygruppe und
$R^2$ ist $NR^a$-$(CH_2)_n$-$NR^aR^b$
mit n = 0-12, bevorzugt mit n = 0 oder 2-5, wobei $R^a$ und $R^b$ jeweils Wasserstoff, $C_1$-$C_4$-Alkyl oder Benzyl sein können, oder
$R^2$ ist $NR^a$-$CHR^3$-$CH_2$-$R^4$,
wobei $R^a$ die genannte Bedeutung hat und $R^3$ Wasserstoff oder eine Carboxylgruppe und $R^4$ den Rest einer L-Aminosäure oder eines biogenen Amins, bevorzugt den Rest einer aromatischen L-Aminosäure oder eines aromatischen biogenen Amins und besonders bevorzugt den Rest des biogenen Amins Tyramin, Tryptamin, Histamin, Dopamin oder 5-Hydroxydopamin darstellt, wobei die L-Aminosäure durch $R^2$ mit $R^3$ = Carboxyl und das biogene Amin durch $R^2$ mit $R^3$ = Wasserstoff definiert ist.

Von den durch chirurgische bzw. strahlentherapeutische Maßnahmen nicht mehr heilbaren malignen Tumoren sind derzeit etwa 7 bis 10 % durch Chemotherapie heilbar. Insbesondere weisen schnell proliferierende Tumoren, wie z. B. die akute lymphatische Leukämie des Kindes, der Morbus Hodgkin, Hodentumoren und Wilmstumoren eine hohe Sensitivität gegen Chemotherapie mit Remissionsraten von 60 bis 90 % auf.

Weiterhin kann durch Chemotherapie bei ca. 30 % aller Tumorpatienten eine Verlangsamung des Tumorwachstums bzw. eine partielle oder komplette Remission der Tumorerkrankungen erreicht werden. Nach unterschiedlich langen Zeitintervallen tritt jedoch auch bei Fortsetzung der Chemotherapie ein Rezidiv des Tumors auf, der dann zumeist gegen die zunächst erfolgreiche Chemotherapie resistent ist. Diese unter Chemotherapie auftretende sogenannte "sekundäre Resistenz" begrenzt dann die Möglichkeit einer weiteren chemotherapeutischen Beeinflussung des Tumors. Zu dieser Gruppe von Tumoren gehören die Mamma- und Ovarialtumoren, die kleinzelligen Lungenkarzinome sowie ein Teil der Lymphome.

Viele maligne Erkrankungen lassen sich derzeit jedoch durch Chemotherapie nicht oder nur marginal beeinflussen, so daß in diesen Fällen von einer primären Resistenz des Tumors gegen die heute bekannten Substanzen ausgegangen werden muß. In diese Gruppe der Tumoren gehören die nicht kleinzelligen Lungentumoren sowie der große Teil der Gastrointestinaltumoren. Betrachtet man diese Gruppe der primär resistenten Tumoren unter dem Aspekt ihrer Proliferationsgeschwindigkeit, so fällt auf, daß gerade die schwer oder nicht durch Chemotherapie beeinflußbaren Tumoren eine besonders langsame Proliferationsrate aufweisen.

Mit höchster Priorität gilt es daher nach neuen Substanzen zu suchen, die gerade bei langsam proliferierenden, primär resistenten Humantumoren wirksam sind. Auch sollten zukünftige Chemotherapeutika möglichst keine Kreuzresistenz zu bereits bekannten Substanzen aufweisen, um mit Aussicht auf Erfolg auch bei vorliegender sekundärer Resistenz gegen bereits etablierte Substanzen einsetzbar zu sein.

Aus der Klasse der Anthracycline besitzen besonders Doxorubicin (Adriamycin), Epirubicin oder Daunomycin bei einer Vielzahl von Tumorerkrankungen therapeutische Wirksamkeit. Jedoch wird nach

mehrmaliger Behandlung eine deutliche Verringerung der antitumoralen Wirksamkeit bis hin zur Wirkungslosigkeit beobachtet, bedingt durch eine sich aufbauende "sekundäre Resistenz" der Tumorzellen gegen das Anthracyclin.

Bei der tumortherapeutischen Verwendung dieser bekannten Anthracycline liegt ein weiteres wesentliches Problem darin, daß diese Verbindungen neben der gewünschten zytostatischen Wirksamkeit auch unerwünschte Nebenwirkungen aufweisen, wie beispielsweise eine hämatologische oder cardiale Toxizität.

Die tumortherapeutische Wirksamkeit von Daunomycin und Adriamycin wird vor allem auf die Fähigkeit dieser Anthracycline zur Interkalation in die DNA zurückgeführt, was das Wachstum der Tumorzelle unterbindet und den Zelltod herbeiführt.

Ausgehend von diesem Stand der Technik ist es Aufgabe der vorliegenden Erfindung, neue Anthracyclinderivate zu schaffen, die möglichst nicht kreuzresistent gegenüber Adriamycin sind und die sich durch ein neues Wirkungsspektrum und eine im Vergleich zu Adriamycin geringere Toxizität auszeichnen und somit vorteilhafte Anwendung in der Tumortherapie finden können.

Zu diesem Zweck wurde bereits vorgeschlagen, Rhodosaminyl-Anthracyclinone der Formel I zu synthetisieren, in denen $R^1$ die genannte Bedeutung hat und $R^2$ Hydroxy, $C_1$-$C_4$-Alkoxy, verzweigt oder unverzweigt oder substituiert oder unsubstituiert, oder $NR^a_2$ mit $R^a$ = Wasserstoff oder $C_1$-$C_4$-Alkyl ist.

Es wurden nun die Anthracyclinderivate der Formel I gefunden, bei denen gegenüber den Anthracyclinen des Standes der Technik bei Variation des Restes $R^2$ in der durch die Definition von $R^2$ angegebenen Art und Weise die Wasserlöslichkeit und/oder Reaktivität und/oder Toxizität des Anthracyclins verändert ist.

Beispielsweise wurde gefunden, daß, wenn $R^2$ $NH$-$(CH_2)_n$-$NH_2$ mit n = 2 oder 4 ist, das Anthracyclin-Molekül amphotere Eigenschaften aufweist und seine Wasserlöslichkeit im Vergleich zu den Anthracyclinen des Standes der Technik erhöht ist.

Ferner wurde gefunden, daß, wenn $R^2$ $NH$-$CHR^3$-$CH_2$-$R^4$ ist, worin $R^3$ Wasserstoff und $R^4$ der Rest des biogenen Amins Histamin, Tyramin oder Tryptamin ist, das Anthracyclin - neben seiner Fähigkeit zur Interkalation in die DNA - auch mit bestimmten Enzymen interagieren kann, was seine Reaktivität im Vergleich zu den Anthracyclinen des Standes der Technik verändert.

Gegenstand der Erfindung sind daher Verbindungen der Formel I mit den angegebenen Bedeutungen.

Bevorzugte Bedeutungen:

$R^2$ ist $NR^a$-$(CH_2)_n$-$NR^aR^b$;

$R^2$ ist $NR^a$-$NR^aR^b$, $NR^a$-$(CH_2)_2$-$NR^aR^b$, oder $NR^a$-$(CH_2)_4$-$NR^aR^b$, wobei Derivate mit $R^a$ = $R^b$ = Wasserstoff bevorzugt sind;

$R^2$ ist $NR^a$-$CHR^3$-$CH_2$-$R^4$, wobei die Verbindungen, in welchen $R^3$ Wasserstoff ist, bevorzugt sind;

$R^4$ ist der Rest einer aromatischen L-Aminosäure oder eines aromatischen biogenen Amins;

$R^4$ ist der Rest des biogenen Amins Tyramin, Tryptamin, Histamin, Dopamin oder 5-Hydroxydopamin.

Das Verfahren zur Herstellung der neuen erfindungsgemäßen zytostatisch wirksamen Anthracyclinderivate der Formel I besteht darin, daß man eine Verbindung der Formel I, in welcher $R^1$ die genannte Bedeutung hat und $R^2$ $C_1$-$C_4$-Alkoxy, verzweigt oder unverzweigt, oder Benzyloxy ist, mit einer Verbindung der Formel $HR^aN$-$(CH_2)_n$-$NR^aR^b$, worin n, $R^a$ und $R^b$ die genannte Bedeutung haben, oder mit einem biogenen Amin oder einer Aminosäure in einem geeigneten Lösungsmittel, wie beispielsweise Dimethylformamid oder Ethanol, bei einer Temperatur zwischen beispielsweise 0 °C und der Siedetemperatur des Lösungsmittels zur Reaktion bringt und die so erhaltene Verbindung der Formel I, in welcher $R^1$ die genannte Bedeutung hat und $R^2$ $NR^a$-$(CH_2)_n$-$NR^aR^b$ oder $NH$-$CHR^3$-$CH_2$-$R^4$ ist, wobei n, $R^a$, $R^b$, $R^3$ und $R^4$ die genannte Bedeutung haben, isoliert und aufreinigt.

Die nach dem erfindungsgemäßen Verfahren erhaltenen neuen Anthracyclinderivate zeichnen sich durch zytostatische Aktivität aus, und sie können daher zusammen mit den üblichen pharmazeutischen Konfektionierungs- und/oder Verdünnungsmitteln zu Arzneimitteln verarbeitet werden, die in der Krebstherapie Anwendung finden. Die Dosierungs- und Anwendungsweise entspricht dabei im wesentlichen derjenigen für die bekannten Substanzen Adriamycin, Daunomycin, Aclacinomycin, 4′-epi-Adriamycin, 4′-Methoxyadriamycin oder 4′-Desoxyadriamycin.

Die solchermaßen hergestellten Arzneimittel können zusätzlich noch andere Wirkstoffe enthalten, sofern diese zusammen mit den erfindungsgemäßen Verbindungen keine unerwünschten Nebenwirkungen zeigen.

Die zytostatische Wirksamkeit der erfindungsgemäßen Verbindungen wurde anhand von L1210 Leukämiezellen der Maus getestet. Hierzu wurde die Koloniebildung von L1210 Leukämiezellen in Agarplatten herangezogen. Diese Methode dient zum Nachweis des Einflusses der Testsubstanzen auf das Wachstumsverhalten der Zellen über 1 Stunde oder über mehrere Generationen. Bei einer Zellzykluszeit von 10-12 Stunden werden dabei in der Testzeit von 7 Tagen etwa 14 aufeinanderfolgende Generationen beobachtet. Die erfindungsgemäßen zytostatisch wirksamen Substanzen bewirken in diesem Test eine Reduktion der zu beobachtenden Koloniezahl gegenüber einer unbehandelten Kontrollprobe.

Einzelheiten des angewandten Testverfahrens ergeben sich aus den nachfolgenden Beschreibungen der Verfahrensweisen zur Ermittlung der Koloniebildung.

Zur Erläuterung des erfindungsgemäßen Herstellungsverfahrens werden nachfolgend Beispiele 1 bis 7 aufgeführt, in denen bevorzugte erfindungsgemäße Verbindungen nach den beanspruchten Verfahren hergestellt wurden.

Charakterisierung von Verbindungen der Formel I

Der Verlauf der Reaktionen sowie die resultierenden Verbindungen wurden dünnschichtchromatographisch oder mit der HPLC-Technik untersucht. Dünnschichtchromatographie erfolgte, sofern nicht anders vermerkt, auf Kieselgel-Fertigplatten (Merck). Säulenchromatographie erfolgte über Kieselgel 60 (Merck) der Korngröße 0.040-0.063 mm. Die Ausbeuten sind nicht optimiert.

Für die Dünnschicht- und Säulenchromatographie wurden folgende Laufmittelgemische verwendet (Angaben jeweils in Volumenprozent):

| Laufmittelgemische Zusammensetzung | A | B | C |
|---|---|---|---|
| Chloroform (%) | 70 | 89 | 77 |
| Methanol (%) | 18 | 7.4 | 14 |
| Essigsäure (%) | 8.5 | 3 | 7 |
| Wasser (%) | 3.5 | 0.6 | 2 |

Die Strukturen der hergestellen Verbindungen wurden mittels $^1H$-NMR- sowie MS-Spektroskopie ermittelt.

BEISPIELE

Herstellung der Ausgangsverbindungen:

Herstellung der Ausgangsverbindung der Formel II, in welcher $R^1$ Wasserstoff und $R^2$ Ethoxy ist:

7-0-(3′-N-Ethoxycarbonylmethyl-3′-N-methyl-α-L-daunosaminyl)-β-rhodomycinon

100 mg (0.189 mmol) 7-0-(3′-N-Methyl-α-L-daunosaminyl)-β-rhodomycinon und 75 µl (113 mg = 0.677 mmol = 3.58 equiv.) Bromessigsäureethylester wurden in Gegenwart von 80 µl (58 mg = 0.574 mmol = 3.0 equiv.) Triethylamin während 2 h umgesetzt. Das Produktgemisch wurde nach der Einengung unverzüglich in wenig Chloroform gelöst, auf eine in Ether angesetzte Kieselgel-Säule (15 g Kieselgel) aufgetragen und zur Entfernung des überschüssigen Bromessigesters mit ca. 100 ml Ether eluiert. Die gewünschte Verbindung wurde nachfolgend in Chloroform/Ethanol (20/1) ($R_F$ 0.32) eluiert.
Ausbeute 70 mg (0.114 mmol) = 60 %
MS-FAB $(M+H^+)$ m/e = 616
$^1H$ NMR (300 MHz, $CDCl_3$) δ 1.12 (t, 3H, $J_{13,14}$ = 7.4 Hz, Me-14), 1.16 (t, 3H, $J_{b,c}$ = 7.1 Hz, Me-c), 1.40 (d, 3H, $J_{5',6'}$ = 6.8 Hz, Me-6′), 1.7-1.9 (m, 4H, $CH_2$-2′ und $CH_2$-13), 2.11 (dd, IH, $J_{7,8a}$ = 4 Hz, $J_{8a,8b}$ = 15 Hz, H-8a), 2.26 (d, 1H, $J_{8a,8b}$ = 15 Hz, H-8b), 2.38 (s, 3H, N-$CH_3$), 2.72 (d, 1H, $J_{10,OH-10}$ = 4 Hz, OH-10), 2.82 (m, 1H, H-3′), 3.14 (bs, 1H, OH-4′), 3.31 (d, 1H, $J_{a,a'}$ = 17 Hz, H-a), 3.33 (d, 1H, $J_{a,a'}$ = 17 Hz, H-a′), 3.65 (bs, 1H, H-4′), 4.03 (s, 1H, OH-9), 4.08 (m, 3H, $CH_2$-b und H-5′), 4.92 (d, 1H, $J_{10,OH-10}$ = 4 Hz, H-10), 5.15 (m, 1H, H-7), 5.51 (bd, 1H, $J_{1',2'}$ = 2.5 Hz, H-1′), 7.33 (dd, 1H, $J_{1,3}$ = 1 Hz, $J_{2,3}$ = 8.4 Hz, H-3), 7.73 (t, 1H, $J_{1,2}$ = $J_{2,3}$ = 8.4 Hz, H-2), 7.90 (dd, 1H, $J_{1,2}$ = 8.4 Hz, $J_{1,3}$ = 1 Hz, H-1), 12.16 (bs, 1H, OH-4), 12.84 (bs, 1H, OH-6), 13.63 (bs, 1H, OH-11).

Beispiel 1:

Herstellung einer Verbindung der Formel I, in welcher $R^1 = H$, und $R^2 = NH\text{-}NH_2$ ist:

7-0-(3′-N-Hydrazinocarbonylmethyl-3′-N-methyl-α-L-daunosaminyl)-β-rhodomycinon (Verbindung 1):

Eine Lösung von 7-0-(3′-N-Ethoxycarbonylmethyl-3′-N-methy-lα-L-daunosaminyl)-β-rhodomycinon (154 mg = 0.25 mmol) in Ethanol (38 ml) wurde mit 100 % Hydrazinhydrat (17,5 µl = 18 mg = 0,36 mmol = 1,44 equiv.) versetzt und 60 h bei Raumtemperatur im Dunkeln gerührt. Dann wurde am Rotationsverdampfer zur Trockne eingeengt und das Reaktionsgemisch über 16 g Kieselgel im Laufmittelgemisch A getrennt ($R_F$ 0,26). Die vereinigten Fraktionen wurden zur Phasentrennung mit Wasser versetzt, mit 10-proz. (w/v) Natronlauge auf pH 7 gebracht und danach durch Zusatz gesättigter wäßriger Natriumhydrogencarbonat-Lösung auf pH 8 eingestellt. Dann wurden die Phasen im Scheidetrichter getrennt, die wäßrige Phase mehrmals mit Chloroform extrahiert und die vereinigten organischen Phasen am Rotationsverdampfer zur Trockne eingeengt.
Ausbeute 51 mg (0,085 mmol) = 34 %
MS-FAB $(M+H^+)$ m/e = 602
$^1H$ NMR (300 MHz, $CDCl_3/D_6$-DMSO (6/1)) δ 1.10 (t, 3H, $J_{13,14}$ = 7.4 Hz, Me-14), 1.35 (d, 3H, $J_{5',6'}$ = 6.5 Hz, Me-6′), 1.6-2.0 (m, 4H, $CH_2$-2′ und $CH_2$-13), 2.19 (bs, 2H, $CH_2$-8), 2.26 (s, 3H, N-$CH_3$), 2.52 (bd, 1H, $J_{2',3'}$ = 10,5 Hz, H-3′), 3.06 (d, 1H, $J_{a,a'}$ = 16.6 Hz, H-a), 3.19 (d, 1H, $J_{a,a'}$ = 16.6 Hz, H-a′), 3.70 (bs, 1H, H-4′), 3.90 (bs, 1H, OH-9), 4.06 (q, 1H, $J_{5',6'}$ = 6.6 Hz, H-5′), 4.84 (s, 1H, H-10), 5.12 (m, 1H, H-7), 5.48 (bd, 1H, $J_{1',2'}$ = 3.2 Hz, H-1′), 7.31 (dd, 1H, $J_{1,3}$ = 1 Hz, $J_{2,3}$ = 8.4 Hz, H-3), 7.72 (t, 1H, $J_{1,2}$ = $J_{2,3}$ = 8.3 Hz, H-2), 7.87 (dd, 1H, $J_{1,2}$ = 7.7 Hz, $J_{1,3}$ = 1 Hz, H-1), 9.03 (s, 1H, Hydrazid-NH).

Beispiel 2:

Herstellung der Verbindung der Formel I, in welcher $R^1$ = H und $R^2$ = $NH\text{-}(CH_2)_2\text{-}NH_2$ ist:

7-0-(3′-N-Ethylendiaminocarbonylmethyl-3′-N-methyl-α-L-daunosaminyl)-β-rhodomycinon (Verbindung 2):

Eine Lösung von 7-O-(3′-N-Ethoxycarbonylmethyl-3′-N-methyl-α-L-daunosaminyl)-β-rhodomycinon (150 mg = 0.24 mmol) in Dimethylformamid (20 ml) wurde mit 1 ml Ethylendiamin versetzt und 3 h bei Raumtemperatur im Dunkeln gerührt. Dann wurde am Rotationsverdampfer zur Trockne eingeengt, das Reaktionsgemisch über 15 g Kieselgel im Laufmittelgemisch A getrennt ($R_F$ 0,15) und die Fraktionen analog Beispiel 1 aufgearbeitet.
Ausbeute 120 mg (0,19 mmol) = 79 %
MS-FAB $(M+H^+)$ m/e = 630
$^1H$ NMR (300 MHz, $CDCl_3/D_6$-DMSO (4/1)) δ 1,09 (t, 3H, $J_{13,14}$ = 7.4 Hz, Me-14), 1.30 (d, 3H, $J_{5',6'}$ = 6.5 Hz, Me-6′), 1.6-1.9 (m, 4H, $CH_2$-2′ und $CH_2$-13), 2.19 (bs, 2H, $CH_2$-8), 2.28 (s, 3H, N-$CH_3$), 2.55 (m, 1H, H-3′), 2.84 (m, 2H, $CH_2$-c), 3.01 (d, 1H, $J_{a,a'}$ = 18 Hz, H-a), 3.15 (d, 1H, $J_{a,a'}$ = 18 Hz, H-a′), 3.25 (m, 1H, H-b), 3.38 (m, 1H, H-b′), 3.62 (bs, 1H, H-4′), 4.05 (q, 1H, $J_{5',6'}$ = 6.6 Hz, H-5′), 4.82 (s, 1H, H-10), 5.12 (m, 1H, H-7), 5.48 (bd, 1H, $J_{1',2'}$ = 3 Hz, H-1′), 7.32 (dd, 1H, $J_{1,3}$ = 1 Hz, $J_{2,3}$ = 8.4 Hz, H-3), 7.73 (t, 1H, $J_{1,2}$ = $J_{2,3}$ = 8 Hz, H-2), 7.87 (dd, 1H, $J_{1,2}$ = 7.5 Hz, $J_{1,3}$ = 1 Hz, H-1), 8.06 (t, 1H, $J_{NH,b}$ = 6 Hz, -CO-NH-).

Beispiel 3:

Herstellung einer Verbindung der Formel I, in welcher $R^1$ = H und $R^2$ = $NH\text{-}(CH_2)_2\text{-}N(CH_3)_2$ ist:

7-0-(3′-N-(Dimethylaminoethylaminocarbonyl)-3′-N-methyl-α-L-daunosaminyl)-β-rhodomycinon (Verbindung 3):

Eine Losung von 7-O-(3′-N-Ethoxycarbonylmethyl-3′-N-methyl-α-L-daunosaminyl)-β-rhodomycinon (16 mg = 0.026 mmol) in Methanol (8 ml) wurde mit 100 µl (81 mg = 0.92 mmol) N,N-Dimethylaminoethylamin versetzt und 16 h bei Raumtemperatur im Dunkeln gerührt. Dann wurde am Rotationsverdampfer zur Trockne eingeengt und im Hochvakuum getrocknet. Das Reaktionsgemisch wurde über 4 g Kieselgel im Laufmittelgemisch A getrennt ($R_F$ 0,14) und die Fraktionen analog Beispiel 1 aufgearbeitet.
Ausbeute 13 mg (0,020 mmol) = 77 %
$^1$H NMR (300 MHz, $CDCl_3$) δ 1.12 (t, 3H, $J_{13,14}$ = 7.4 Hz, Me-14), 1.30 (d, 3H, $J_{5',6'}$ = 6.4 Hz, Me-6′), 1.7-2.05 (m, 4H, $CH_2$-2′ und $CH_2$-13), 2.11 (dd, 1H, $J_{7,8a}$ = 4 Hz, $J_{8a,8b}$ = 15 Hz, H-8a), 2.25 (d, 1H, $J_{8a,8b}$ = 15 Hz, H-8b), 2.28 (s, 6H, $N(CH_3)_2$), 2.31 (s, 3H, N-$CH_3$), 2.51 (m, 3H, $CH_2$-c und H-3′), 2.98 (d, IH, $J_{a,a'}$ = 18 Hz, H-a), 3.19 (m, 1H, H-b), 3.20 (d, 1H, $J_{a,a'}$ = 18 Hz, H-a′), 3.68 (m, 1H, H-b′), 5.04 (q, 1H, $J_{5',6'}$ = 6.5 Hz, H-5′), 4.10 (s, 1H, OH-9), 4.91 (s, 1H, H-10), 5.15 (m, 1H, H-7), 5.52 (bd, 1H, $J_{1',2'}$ = 3 Hz, H-1′), 7.33 (d, 1H, $J_{2,3}$ = 8 Hz, H-3), 7.72 (t, 1H, $J_{1,2}$ = $J_{2,3}$ = 8 Hz, H-2) 7.89 (d, 1H, $J_{1,2}$ = 7 Hz, H-1), 8.14 (m, 1H, NH).

Beispiel 4:

Herstellung einer Verbindung der Formel I, in welcher $R^1$ = H und $R^2$ = NH-$(CH_2)_4$-$NH_2$ ist:

7-0-(3′-N-(4-Aminobutylaminocarbonyl)-3′-N-methyl-α-L-daunosaminyl)-β-rhodomycinon (Verbindung 4):

Eine Lösung von 7-O-(3′-N-Ethoxycarbonylmethyl-3′-N-methyl-α-L-daunosaminyl)-β-rhodomycinon (16 mg = 0,026 mmol) in Acetonitril (3 ml) wurde mit 200 µl 1,4-Diaminobutan versetzt und 3 h bei Raumtemperatur im Dunkeln gerührt. Dann wurde am Rotationsverdampfer zur Trockne eingeengt, das Reaktionsgemisch über 3 g Kieselgel im Laufmittelgemisch C getrennt ($R_F$ 0,16) und die Fraktionen analog Beispiel 1 aufgearbeitet.
Ausbeute 11 mg (0,017 mmol) = 65 %
MS-FAB ($M+H^+$) m/e = 658
$^1$H NMR (300 MHz, $CDCl_3$/$D_6$-DMSO (4/1)) δ 1.10 (t, 3H, $J_{13,14}$ = 7.4 Hz, Me-14), 1.32 (d, 3H, $J_{5',6'}$ = 6.5 Hz, Me-6′), 1.4-1.6 (m, 4H, $CH_2$-c und $CH_2$-d), 1.6-2.0 (m, 4H, $CH_2$-2′ und $CH_2$-13), 2.17 (bs, 2H, $CH_2$-8), 2.29 (s, 3H, N-$CH_3$), 2.55 (m, 1H, H-3′), 2.69 (bt, 2H, $J_{d,e}$ = 6.5 Hz, $CH_2$-e), 3.06 (d, 1H, $J_{a,a'}$ = 17 Hz, H-a), 3.08 (d, 1H, $J_{a,a'}$ = 17 Hz, H-a′), 3.20 (bt, 2H, $J_{b,c}$ = 6.5 Hz, $CH_2$-b), 3.71 (bs, 1H, H-4′), 4.06 (q, 1H, $J_{5',6'}$ = 6.6 Hz, H-5′), 4.82 (s, 1H, H-10), 5.12 (m, 1H, H-7), 5.48 (bd, 1H, $J_{1',2'}$ = 3 Hz, H-1′), 7.29 (dd, 1H, $J_{1,3}$ = 1 Hz, $J_{2,3}$ = 8.4 Hz, H-3), 7.70 (t, 1H, $J_{1,2}$ = $J_{2,3}$ = 8 Hz, H-2), 7.85 (dd, 1H, $J_{1,2}$ = 7.5 Hz, $J_{1,3}$ = 1 Hz, H-1).

Beispiel 5:

7-O-(3′-N-Histaminocarbonylmethyl-3′-N-methyl-α-L-daunosaminyl)-β-rhodomycinon (Verbindung 5)

Eine Lösung von 7-O-(3′-N-Ethoxycarbonylmethyl-3′-N-methyl- α-L-daunosaminyl)-β-rhodomycinon (25 mg = 0,04 mmol) in Dimethylformamid (7 ml) wurde mit 20 mg (0,18 mmol) Histamin versetzt und 30 h bei 50 °C im Dunkeln gerührt. Dann wurde am Rotationsverdampfer zur Trockne eingeengt, das Reaktionsgemisch im Laufmittelgemisch C getrennt ($R_F$ 0,14) und die Fraktionen analog Beispiel 1 aufgearbeitet.
Ausbeute 21 mg (0,03 mmol) = 75 %
MS-FAB ($M+H^+$) m/e = 681
$^1$H NMR (300 MHz, $CDCl_3$) δ 1,13 (t, 3H, $J_{13,14}$ = 7.4 Hz, Me-14), 1.40 (d, 3H, $J_{5',6'}$ = 6.5 Hz, Me-6′), 1.6-2.0 (m, 4H, $CH_2$-2′ und $CH_2$-13), 2.11 (dd, 1H, $J_{7,8a}$ = 4 Hz, $J_{8a,8b}$ = 15 Hz, H-8a), 2.26 (d, 1H, $J_{8a,8b}$ = 15 Hz, H-8b), 2.29 (s, 3H, N-$CH_3$), 2.30 (bd, 1H, $J_{2',3'}$ = 11 Hz, H-3′), 2.78 (bt, 2H, $J_{b,c}$ = 5.6 Hz, $CH_2$-c), 2.96 (d, 1H, $J_{a,a'}$ = 17 Hz, H-a), 3.14 (d, 1H, $J_{a,a'}$ = 17 Hz, H-a′), 3.49 (m, 2H, $CH_2$-b), 3.73 (s, 1H, H-4′), 4.10 (q, 1H, $J_{5',6'}$ = 6.3 Hz, H-5′), 4.92 (s, 1H, H-10), 5.16 (m, 1H, H-7), 5.51 (bd, 1H, $J_{1',2'}$ = 3.5 Hz, H-1′), 6.81 (s, 1H, H-d), 7.33 (dd, 1H, $J_{1,3}$ = 1 Hz, $J_{2,3}$ = 8.4 Hz, H-3), 7.56 (s, 1H, H-e), 7.73 (t, 1H, $J_{1,2}$ = $J_{2,3}$ = 8 Hz, H-2), 7.89 (dd, 1H, $J_{1,2}$ = 7.5 Hz, $J_{1,3}$ = 1 Hz, H-1), 8.57 (bs, 1H, NH).

Beispiel 6:

7-O-(3′-N-Methyl-3′-N-tyraminocarbonylmethyl-α-L-daunosaminyl)-β-rhodomycinon (Verbindung 6)

Die Umsetzung erfolgte analog Beispiel 5 mit 20 mg (0,032 mmol) Ausgangsverbindung und 20 mg (0,15 mmol) Tyramin in Dimethylformamid (3 ml), wobei jedoch 50 h bei 50-60° C gerührt wurde. Die Reinigung erfolge in Laufmittelgemisch C ($R_F$ 0,5).
Ausbeute 12 mg (0,017 mmol) = 53 %
MS-FAB ($M+H^+$) m/e = 707
$^1$H NMR (300 MHz, $CDCl_3/D_6$-DMSO (4/1)) δ 1.10 (t, 3H, $J_{13,14}$ = 7.4 Hz, Me-14), 1.32 (d, 3H, $J_{5',6'}$ = 6.5 Hz, Me-6′), 1.6-2.0 (m, 4H, $CH_2$-2′ und $CH_2$-13), 2.19 (bs, 2H, $CH_2$-8), 2.20 (s, 3H, N-$CH_3$), 2.54 (bd, 1H, $J_{2',3'}$ = 11 Hz, H-3′), 2.69 (bt, 2H, $J_{b,c}$ = 7 Hz, $CH_2$-c), 3.03 (d, 1H, $J_{a,a'}$ = 17 Hz, H-a), 3.05 (d, 1H, $J_{a,a'}$ = 17 Hz, H-a′), 3.40 (m, 2H, $CH_2$-b), 3.65 (bs, 1H, H-4′), 3.89 (bs, 1H, OH-9), 4.05 (q, 1H, $J_{5',6'}$ = 6.7 Hz, H-5′), 4.85 (s, 1H, H-10), 5.12 (m, 1H, H-7), 5.47 (bd, 1H, $J_{1',2'}$ = 3 Hz, H-1′), 6.74 (d, 2H, $J_{d,e}$ = 8.5 Hz, $CH_2$-d), 6.97 (d, 2H, $J_{d,e}$ = 8.5 Hz, $CH_2$-e), 7.32 (dd, 1H, $J_{1,3}$ = 1 Hz, $J_{2,3}$ = 8.4 Hz, H-3), 7.49 (bt, 1H, $J_{NH,b}$ = 6 Hz, NH), 7.72 t (1H, $J_{1,2}$ = $J_{2,3}$ = 8 Hz, H-2), 7.87 (dd, 1H, $J_{1,2}$ = 7.5 Hz, $J_{1,3}$ = 1 Hz, H-1).

Beispiel 7:

7-O-(3′-N-Methyl-3′-N-tryptaminocarbonylmethyl-α-L-daunosaminyl)-β-rhodomycinon (Verbindung 7)

Die Umsetzung erfolgte analog Beispiel 5 mit 20 mg (0,032 mmol) Ausgangsverbindung und 20 mg (0,12 mmol) Tryptamin in Dimethylformamid (3 ml), wobei jedoch 50 h bei 50-60° C gerührt wurde. Die Reinigung erfolgte im Laufmittelgemisch C ($R_F$ 0,46).
Ausbeute 17 mg (0,023 mmol) = 72 %
MS-FAB ($M+H^+$) m/e = 730
$^1$H NMR (300 MHz, $CDCl_3$) δ 1.13 (t, 3H, $J_{13,14}$ = 7.4 Hz, Me-14), 1.23 (d, 3H, $J_{5',6'}$ = 6.5 Hz, Me-6′), 1.5-2.0 (m, 4H, $CH_2$-2′ und $CH_2$-13), 2.12 (dd, 1H, $J_{7,8a}$ = 4 Hz, $J_{8a,8b}$ = 15 Hz, H-8a), 2.17 (s, 3H, N-$CH_3$), 2.20 (d, 1H, $J_{8a,8b}$ = 15 Hz, H-8a′), 2.48 (m, 1H, H-3′), 2.96 (bt, 2H, $J_{b,c}$ = 6.5 Hz, $CH_2$-c), 3.46 (bs, 1H, H-4′), 3.59 (m, 2H, $CH_2$-b), 3.96 (s, 1H, OH-9), 4.00 (q, 1H, $J_{5',6'}$ = 6.3 Hz, H-5′), 4.93 (s, 1H, H-10), 5.11 (m, 1H, H-7), 5.41 (bs, 1H, H-1′), 7.0 (d, 1H, $J_{d,NH}$ = 2.3 Hz, H-d), 7.08 (dt, 1H) und 7.17 (dt, 1H) ($J_{e,f}$ = $J_{f,g}$ = $J_{g,h}$ = 7 Hz, $J_{e,g}$ = $J_{f,h}$ = 1 Hz, H-f und H-g), 7.32 (dd, 1H, $J_{1,3}$ = 1 Hz, $J_{2,3}$ = 8.4 Hz, H-3), 7.33 (d, 1H, $J_{e,f}$ = 8.0 Hz, H-e), 7.54 (d, 1H, $J_{g,h}$ = 7.7 Hz, H-h), 7.72 (t, 1H, $J_{1,2}$ = $J_{2,3}$ = 8 Hz, H-2), 7.88 (dd, 1H, $J_{1,2}$ = 7.5 Hz, $J_{1,3}$ = 1 Hz, H-1), 8.29 (bs, 1H, CO-NH).

Zytotoxizität von Verbindungen der Formel I gegen L1210-Leukämiezellen der Maus in vitro

Verfahrensweise zur Ermittlung der Koloniebildung von L1210 Leukämiezellen in Soft-Agar

500 Leukämiezellen pro Platte wurden mit unterschiedlichen Konzentrationen der Testsubstanz 1 Stunde bei 37° C inkubiert. Anschließend wurden die Zellen zweimal mit McCoy5A-Medium gewaschen und schließlich in Petrischalen nach Zugabe von 0,3 % Agar ausgegossen. Kontrollen wurden lediglich mit frischem Medium inkubiert. Anstelle der einstündigen Inkubation wurden in manchen Fällen unterschiedliche Konzentrationen und Testsubstanzen der oberen Agarschicht zugemischt, um so eine kontinuierliche Exposition der Zellen über die gesamte Inkubationszeit zu erreichen. Nach Erstarren des Agars wurden die Platten im Brutschrank 7 Tage bei 37° C inkubiert (5 Vol.-% $CO_2$, 95 % relative Luftfeuchtigkeit). Anschließend wurde die Zahl der entstandenen Kolonien mit einem Durchmesser von mehr als 60 µm gezählt. Die Ergebnisse wurden angegeben als Koloniezahl in behandelten Agarplatten in Prozent der unbehandelten Kontrolle. Aus der so erhaltenen Dosiswirkungskurve wurde die $IC_{50}$ als Maß für die Wirksamkeit der Substanz ermittelt. Die Ergebnisse für die hier beschriebenen Verbindungen im Vergleich zu Adriamycin sind in Tabelle 1 zusammengefaßt.

**Tabelle 1:** Zytotoxizität der hergestellten Verbindungen der Formel I gegen L1210 Leukämiezellen in vitro

| Substanz No. (Beispiel) | $R^1$ | $R^2$ | Dauerinkubation $IC_{50}$ (µg/ml) | 1h-Inkubation $IC_{50}$ (µg/ml) |
|---|---|---|---|---|
| 1 | H | $NH-NH_2$ | 0,32 | 0,21 |
| 2 | H | b c<br>$NH-CH_2-CH_2-NH_2$ | 0,35 | 1,0 |
| 3 | H | b c<br>$NH-CH_2-CH_2-N(CH_3)_2$ | 0,12 | 0,26 |
| 4 | H | b c d e<br>$NH-CH_2-CH_2-CH_2-CH_2-NH_2$ | 0,35 | 1,3 |
| 5 | H | b c<br>$NH-CH_2-CH_2-$ 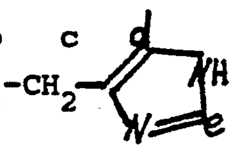 | 0,31 | 1,0 |
| 6 | H | b c<br>$NH-CH_2-CH_2-$ 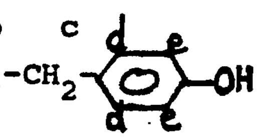 | 0,41 | 1,3 |
| 7 | H | b c<br>$NH-CH_2-CH_2-$ 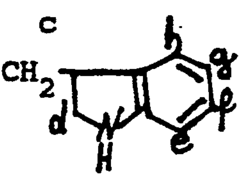 | 0,36 | 1,2 |

**Ansprüche**

1. Neue zytostatisch wirksame Anthracyclinderivate der Formel I

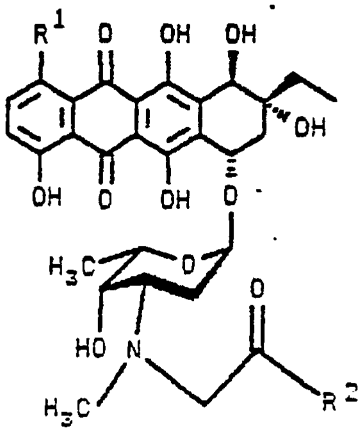

I

und deren physiologisch unbedenklichen Salze , wobei
$R^1$ Wasserstoff oder eine Hydroxygruppe und
$R^2$ $NR^a-(CH_2)_n-NR^aR^b$
mit n = 0-12, bevorzugt mit n = 0 oder 2-5 ist, wobei $R^a$ und $R^b$ jeweils Wasserstoff, $C_1$-$C_4$-Alkyl oder Benzyl sein können, oder
$R^2$ $NR^a-CHR^3-CH_2-R^4$ ist,
wobei $R^a$ die genannte Bedeutung hat und $R^3$ Wasserstoff oder eine Carboxylgruppe und $R^4$ der Rest einer L-Aminosäure oder eines biogenen Amins sein kann.

2. Anthracyclinderivate nach Anspruch 1,
**dadurch gekennzeichnet,**
daß $R^2 \cdot NR^a-(CH_2)_n-R^aR^b$ ist.

3. Anthracyclinderivate nach Anspruch 2,
**dadurch gekennzeichnet,**
daß $R^2$ $NR^a-NR^aR^b$, $NR^a-(CH_2)_2-NR^aR^b$, oder $NRa-(CH_2)_4-NR^aR^b$ ist, wobei Derivate mit $R^a$ = $R^b$ = Wasserstoff bevorzugt sind.

4. Anthracyclinderivate nach Anspruch 1,
**dadurch gekennzeichnet,**
daß $R^2$ $NR^a-CHR^3-CH_2-R^4$ ist, wobei die Verbindungen, in welchen $R^3$ Wasserstoff ist, bevorzugt sind.

5. Anthracyclinderivate nach Anspruch 4,
**dadurch gekennzeichnet,**
daß $R^4$ den Rest einer aromatischen L-Aminosäure oder eines aromatischen biogenen Amins darstellt.

6. Anthracyclinderivate nach Anspruch 5,
dadurch gekennzeichnet,
daß $R^4$ den Rest des biogenen Amins Tyramin, Tryptamin, Histamin, Dopamin oder 5-Hydroxydopamin darstellt.

7. Verfahren zur Herstellung von Anthracyclinderivaten nach Anspruch 1,
**dadurch gekennzeichnet,**
daß man eine Verbindung der Formel I, in welcher $R^1$ die genannte Bedeutung hat und $R^2$ $C_1$-$C_4$-Alkoxy, verzweigt oder unverzweigt, oder Benzyloxy ist, mit einer Verbindung der Formel $HR^aN-(CH_2)_n-NR^aR^b$, worin n, $R^a$ und $R^b$ die genannte Bedeutung haben, oder einem biogenen Amin oder einer Aminosäure in einem geeigneten Lösungsmittel, bevorzugt Dimethylformamid oder Ethanol, bei einer Temperatur zwischen 0 °C und der Siedetemperatur des Lösungsmittels zur Reaktion bringt und die so erhaltene Verbindung der Formel I, in welcher $R^1$ die genannte Bedeutung hat und $R^2$ $NR^a-(CH_2)_n-NR^aR^b$ oder $NR^a-CHR^3-CH_2-R^4$ ist, wobei n, $R^a$, $R^b$, $R^3$ und $R^4$ die genannte Bedeutung haben, isoliert und aufreinigt.

8. Anthracyclinderivat nach Anspruch 1 als Arzneimittel.

Patentanspruch für folgende Vertragsstaaten: ES, GR

1. Verfahren zur Herstellung von neuen zytostatisch wirksamen Anthracyclinderivaten der Formel I

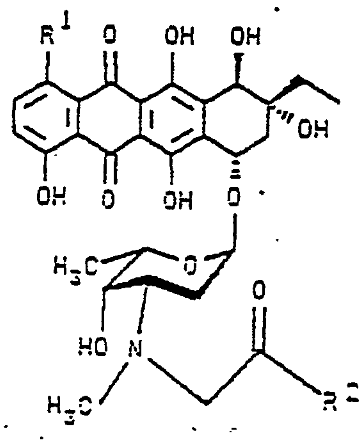

I

und deren physiologisch unbedenklichen Salze, wobei
$R^1$ Wasserstoff oder eine Hydroxygruppe und
$R^2$ $NR^a$-$(CH_2)_n$-$NR^aR^b$
mit n = 0-12, bevorzugt mit n = 0 oder n = 2-5, ist, wobei $R^a$ und $R^b$ jeweils Wasserstoff, $C_1$-$C_4$-Alkyl oder Benzyl sein können, oder
$R^2$ $NR^a$-$CHR^3$-$CH_2$-$R^4$ ist,
wobei $R^a$ die genannte Bedeutung hat und $R^3$ Wasserstoff oder eine Carboxylgruppe und $R^4$ der Rest einer L-Aminosäure oder eines biogenen Amins sein kann,
dadurch gekennzeichnet, daß man eine Verbindung der Formel I, in welcher $R^1$ die genannte Bedeutung hat und $R^2$ $C_1$-$C_4$-Alkoxy, verzweigt oder unverzweigt, oder Benzyloxy ist, mit einer Verbindung der Formel $HR^a$-$(CH_2)_n$-$NR^aR^b$, worin n, $R^a$, $R^b$ die genannte Bedeutung haben, oder einem biogenen Amin oder einer Aminosäure in einem geeigneten Lösungsmittel, bevorzugt Dimethylformamid oder Ethanol, bei einer Temperatur zwischen 0 °C und der Siedetemperatur des Lösungsmittels zur Reaktion bringt und die so hergestellte Verbindung der Formel I, in welcher $R^1$ die genannte Bedeutung hat und $R^2$ $NR^a$-$(CH_2)_n$-$NR^aR^b$ oder $NR^a$-$CHR^3$-$CH_2$-$R^4$ ist, wobei n, $R^a$, $R^b$, $R^3$ und $R^4$ die genannte Bedeutung haben, isoliert und aufreinigt.